(19) Europäisches Patentamt
European Patent Office
Office européen des brevets

(11) Publication number: **0 278 787 B1**

(12) **EUROPEAN PATENT SPECIFICATION**

(45) Date of publication of patent specification: **20.10.93**

(51) Int. Cl.⁵: **C12P 21/00**, C12P 21/02, C07K 1/00

(21) Application number: **88301230.4**

(22) Date of filing: **15.02.88**

The file contains technical information submitted after the application was filed and not included in this specification

(54) **A process for enzymatic production of dipeptides.**

(30) Priority: **13.02.87 DK 725/87**

(43) Date of publication of application: **17.08.88 Bulletin 88/33**

(45) Publication of the grant of the patent: **20.10.93 Bulletin 93/42**

(84) Designated Contracting States: **AT BE CH DE ES FR GB GR IT LI NL SE**

(56) References cited:
**EP-A- 0 017 485**
**EP-A- 0 074 095**
**EP-A- 0 086 053**
**EP-A- 0 099 585**
**US-A- 3 972 773**
**US-A- 4 086 136**

**Carlsberg Res. Commun. Vol. 44, pp. 37-46, 1979**

**Carlsberg Res. Commun. Vol. 45, pp. 237-247, 1980**

**Carlsberg Res. Commun. Vol. 45, pp. 361-367, 1980**

(73) Proprietor: **CARLBIOTECH LTD. A/S**
**Tagensvej 16**
**DK-2200 Copenhagen N(DK)**

(72) Inventor: **Thorbek, Pia**
**Sdr. Jagtvej 39**
**DK-2970 Horsholm(DK)**
Inventor: **Widmer, Fred**
**Bronsholmdalsvej 53**
**DK-2980 Kokkedal(DK)**
Inventor: **Aasmul-Olsen, Stig**
**Skodsborgvej 410, st.tv.**
**DK-2942 Skodsborg(DK)**

(74) Representative: **Christiansen, Ejvind et al**
**HOFMAN-BANG & BOUTARD A/S**
**Adelgade 15**
**DK-1304 Copenhagen K (DK)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.6/3.3.1)

## Description

The present invention concerns a process for enzymatic production of dipeptides and derivatives of dipeptides and of the type stated in the introductory portion of claim 1.

In recent years there has been an increasing interest in dipeptides and dipeptide derivatives optionally containing an amino-acid residue of D-configuration, with a view to their potential pharmacological effects, such as e.g. antibiotics. Likewise, there has been a great interest in dipeptides within fields such as artificial nutrition - human as well as veterinary, sweeteners and within agrochemistry, such as e.g. herbicides.

Such dipeptides H-A-B-Y can be produced by means of known chemical coupling reactions, but all these methods share the feature that, generally, it is necessary to protect the amino acids involved - A and B - on the amino group and the carboxylic acid group, respectively, and frequently also on the side chains if these carry functional groups. Further, there is an inherent risk of side reactions during the chemical coupling step because of the reagents and conditions employed, a major side reaction being racemization, particularly of the A-component. By replacing the chemical coupling step with an enzymatic coupling step proceeding under mild conditions, such side reactions and racemization can be avoided, yielding a stereochemically pure product.

The presence of amino- and carboxyl protective groups is mandatory in chemical coupling as well as in enzymatic coupling using endoproteases, and according to prior knowledge also on the amino function of the substrate in enzymatic exoprotease catalyzed formation of dipeptides.

This adds several undesired features to these processes seriously afflicting their process economy on an industrial scale, particularly apparent in dipeptide synthesis.

The disadvantages are concerned with the introduction of these groups, as well as their removal and presence during process operation, increasing overall process cost and time and affecting overall yield.

Typical examples of amino protective groups commonly used are those of the carbobenzoxy (Z-) and tert-butoxycarbonyl (Boc-) type, which are of a molecular weight comparable to those of the amino acid residues. Firstly, the protective groups will have to be introduced in the starting materials by means of appropriate costly agents in a separate reaction step followed by an isolation step. While present, these hydrophobic groups often have a drastic effect upon the solubility of the intermediates and reaction products, and may afflict both the nature and the amount of solvents required in their processing as well as ease of purification and of deprotection. The deprotection will also take place in a separate step with a following purification step.

For this purpose a series of reactions are available, but with the exception of catalytic hydrogenation, posing industrial problems of its own, these methods are occuring under violent often strongly acidic or basic conditions, frequently giving rise to a series of side reactions, resulting in an impure product or demanding laborious purification.

The last steps in this relatively long series of synthesis steps may thus be a rather comprehensive deprotection to obtain the desired dipeptides, and, owing to the almost inevitable secondary reactions, rather laborious purification procedures are frequently required to provide a product with the desired high purity.

Attempts to avoid amino terminal protection in the production of dipeptides have led to microbial fermentation approaches, like the fermentation process under formation of aspartame described in EP-A1-074095 and EP-A2-102529. This technique is fundamentally different from synthetic approaches and relies on specific organisms for each peptide, and is thus not generally applicable. In addition, yields are often low and recovery from the fermentation broth laborious.

Thus, it is an obvious advantage in terms of overall process economy to be able to avoid protective groups, also on the amino and carboxy termini. It is the object of the present invention to make this possible in the synthesis of dipeptides mediated by serine- and thiol carboxypeptidase catalysis. In some cases, it may be of interest to be able to produce a dipeptide which may carry side-chain protection, but no terminal protection, and it will be shown that it is possible in the process according to this invention, starting from side-chain protected, but amino respectively carboxy unprotected starting materials. In this case, the same advantages of mild reaction conditions and overall process economy may be obtained. If desired, the side-chain protective group may be cleaved by chemical or enzymatic means.

The enzyme catalyzed coupling reactions enabling the use of side-chain unprotected amino acid derivatives and an optionally C-terminal unprotected B-component (nucleophile) are known. See e.g. EP-A-0017485 or the granted equivalent (EP-B1-17485).

EP-B1-17485 describes a process for producing peptides of the general formula

$A_1$-$B_1$-$Z_1$

wherein $A_1$ represents an N-terminal protected L-amino acid residue or an optionally N-terminal protected peptide residue having a C-terminal L-amino acid residue, and $B_1$ represents an L-amino acid residue, and $Z_1$ is OH or a C-terminal protective group, by reaction of a substrate component with an amine component in the presence of an enzyme, and, if desired, cleavage of optional terminal protective groups to provide a peptide of the formula

$A_1$-$B_1$-$Z_1$

which is characterized by reacting a substrate component selected from

amino acid esters, peptide esters, depsipeptides, or optionally N-substituted amino acid amides or peptide amides, or optionally N-terminal protected peptides of the formulae

$A_1$-$OR_1$, $A_1$-$NR_2R_2'$ or $A_1$-$X_1$-OH

wherein $A_1$ is as defined above,
$R_1$ is alkyl, aryl, heteroaryl,aralkyl or an $\alpha$-desamino fragment of an amino acid residue,
$R_2$ and $R_2'$ are each hydrogen, alkyl, aryl, heteroaryl or aralkyl, and
$X_1$ is an L-amino acid residue,

with an amine component (nucleophile) selected from optionally N-substituted L-amino acid amides, L-amino acids or L-amino acid esters, of the formula

H-$B_1$-$NR_3R_3'$ or H-$B_1$-$OR_4$

wherein $B_1$ is as defined above,
$R_3$ and $R_3'$ are each hydrogen, hydroxy, amino, alkyl, aryl, heteroaryl or aralkyl, and
$R_4$ is hydrogen, alkyl, aryl, heteroaryl or aralkyl,

in the presence of an L-specific serine or thiol carboxypeptidase enzyme originating from yeast or of animal, vegetable or other microbial origin, in an aqueous solution or dispersion at pH-5-10.5, preferably at a temperature of 20-50 °C. The preferred enzyme is carboxypeptidase Y from yeast, called CPD-Y in the following. (It is noted that in order to avoid confusion with the present symbol meaning the above-mentioned symbol meanings are not identical with those used in EP-B-17485).

Thus, if a dipeptide is to be produced by the process of EP-B1-17485, the substrate component is necessarily an N-terminal protected amino acid derivative, and the constituent amino acid is necessarily an L-amino acid.

More generally, it is said that the need for N-terminal amino group protection of the substrate component decreases with increasing chain length of the peptide residue and is substantially absent when the peptide residue consists of 3 amino acids, depending, however, upon their type and sequence.

This is illustrated by Breddam et al, "Influence of the substrate structure on carboxypeptidase Y catalyzed peptide bond formation", Carlsberg Res. Commun., vol. 45, p. 361-67, 30th December 1980, in which Ac-Ala-Ala-Ala-OMe and H-Ala-Ala-Ala-OMe were coupled with H-Leu-$NH_2$ in the presence of CPD-Y to form Ac-Ala-Ala-Ala-Leu$NH_2$ and H-Ala-Ala-Ala-Leu$NH_2$ in yields of 90 and 80%, respectively.

Breddam et al also examined the importance of the amino acid configuration for the coupling yield in CPD-Y catalyzed peptide synthesis, cf. the following table, where Ala represents L-alanine and ala represents D-alanine.

| Substrate | Product | Yield (%) |
|---|---|---|
| H-Ala-Ala-Ala-OMe | H-Ala-Ala-Ala-Leu-$NH_2$ | 80 |
| H-Ala-ala-Ala-OMe | H-Ala-ala-Ala-Leu-$NH_2$ | 40 |
| H-Ala-Ala-ala-OMe | H-Ala-Ala-ala-Leu-$NH_2$ | 0 |
| Conditions: 25 mM substrate, 0.1 M KCl, 1 mM EDTA, pH 9.5, CPD-Y = 12 $\mu$m, 0.2 M Leu-NH-$_2$. The reaction was stopped after 20 min. | | |

It appears from the table that if the C-terminal is in D-form, like in H-Ala-Ala-ala-OMe, no peptide synthesis takes place because the ester is not reacted. With the D-amino acid juxtaposed to the C-terminal like in H-Ala-ala-Ala-OMe, reaction takes place, but the coupling yield is reduced to 40% compared with the

80% in the pure L-configuration H-Ala-Ala-Ala-OMe.

Further, it has long been known that some endoproteases can catalyze oligomerization of certain N-unprotected amino acid esters with L-configuration, but it has never been attempted to use this for production of dipeptides which are not simple dimers. Generally, the results of such observations have been a mixture of a series of oligomers, sometimes long, and only in case of product precipitation has it been possible to isolate a single product.

For this reason the use of endoproteases for peptide synthesis has been limited to the use of amino and carboxy terminal protected starting materials, as exemplified by US-A-4.086.136.

The abovementioned amino and carboxy terminal protected starting materials are also mandatory if aspartate endoproteases are used as exemplified by US-A-3.972.773, and if metallo endoproteases are used, as exemplified by the synthesis of Z-AspPheOMe.PheOMe-salt in EP-A1-009585.

Finally, the synthesis of the diastereomeric dipeptides of DL, LD and DD-configuration as well as peptides containing beta-amino acid residues from amino-unprotected starting compounds has so far not been possible with carboxypeptidases nor in general with any proteolytical enzymes (Class EC 3.4). Some efforts have been made with a different class of enzymes, aminoacyl-t-RNA-synthetase (Class EC 6.1) as exemplified by EP-A1-086053. In this case a specific enzyme must be used for each type of amino acid residue, and furthermore, expensive Co-factors like ATP are required. At the same time, yields are very poor, so even though some product was isolated and identified, typically a ten fold excess of Co-factor and a hundred fold excess of nucleophile and up to a thousand fold excess of enzyme by weight was required.

It has now surprisingly been found that the serine and thiol carboxypeptidases used in EP-B1-17485 are capable of utilizing N-unprotected amino acid esters as a substrate component in controlled reactions for synthesis of dipeptides and dipeptide derivatives, and that it is possible to suppress a possible oligomerization of the substrate.

It has moreover surprisingly been found that also N-unprotected amino acid derivatives of D-configuration can be used as substrates in these reactions, so that, in addition to LL-dipeptides, it is also possible to synthesize DL-dipeptides. The reaction rate for D-substrates, however, is generally somewhat lower than for L-substrates under uniform conditions, but, as illustrated below, the difference in rate is much smaller than for the corresponding N-protected amino acid esters, the D-substrate being reacted at a rate which is much smaller than the rate for the L-substrate. The yields are often just as high or higher with the unprotected D-substrates in relation to the unprotected L-substrates as shown by the following examples.

Furthermore, it has even more surprisingly been found that with substrates of D-configuration, nucleophiles of D-configuration can be coupled by means of these enzymes, otherwise known to be L-specific on the amino side of the synthesis point. An amino acid ester incorporated in this manner is not hydrolyzed further.

The process of the invention is thus characterized by the features defined in the characterizing portion of claim 1.

Examples of useful amino acids include aliphatic amino acids, such as monoaminomonocarboxylic acids, e.g. glycine (Gly), alanine (Ala), valine (Val), norvaline (Nval), leucine (Leu), isoleucine (iso-Leu) and norleucine (Nleu), hydroxy amino acids, auch as serine (Ser), threonine (Thr) and homoserine (homo-Ser), sulfur-containing amino acids, such as methionine (Met) or cystine (CysS) and cysteine (CysH), monoaminodicarboxylic acids, such as aspartic acid (Asp), glutamic acid (Glu) and amides thereof, such as asparagine (Asn) and glutamine (Gln), diaminomonocarboxylic acids, such as ornithine (Orn) and lysine (Lys), arginine (Arg), aromatic amino-acids, such as phenylalanine (Phe) and tyrosine (Tyr), as well as heterocyclic amino-acids, such as histidine (His), proline (Pro) and tryptophan (Trp). Examples of useful amino-acids of a more unusual structure are penicillamine (Pen), aminophosphonic acids, such as alanine-phosphonic acid (Alap), aminosulfonic acids, such as taurine (Tau), and $\omega$-amino-acids, such as $\beta$-alanine (BAla). As mentioned, they may be included in D-form in the substrate component and they may also be present in D-form in the nucleophile component.

The advantages of the process of the invention over the given known methods are that minimum or no side-chain protection is required, that no N-protection of the substrate component (which may have both D- and L-configuration) is required, that there is little or no risk of racemization, that there are few synthesis steps, and that a relatively pure end product is obtained. The process therefore provides an extremely simple and economic method of production.

Preferred substrate components are esters in which $R^1$ is a straight or branched alkyl group having 1 to 6 carbon atoms, such as methyl, ethyl, propyl, isopropyl, butyl, isobutyl, tert-butyl, amyl and hexyl, or the aralkyl group benzyl. Particularly preferred nucleophile components are free L-amino-acids or amino-acid amides, in which $R^2$ is H and $R^3$ is H or $C_1$-$C_6$ alkyl, or amino-acid esters in which $R^4$ is a straight or branched alkyl group having 1 to 6 carbon atoms such as any of those given above. More generally, as

defined above, $R^1$ may be alkyl, aryl or aralkyl optionally substituted by inert substituents, e.g. hydroxy or nitro. $R^1$ will usually have no more than 12 carbon atoms, and substituents usually no more than 6.

The invention also comprises the processes involving intermediate formation of a peptide containing the group $-NR^2R^3$ or $-OR^4$, following which this group is cleaved to form a carboxylic acid group. This cleavage may be catalyzed by another or the same enzyme as was used to form the peptide.

Enzymes may also be used to cleave side-chain protective groups. Suitable enzymes are proteolytic enzymes, lipases and esterases, according to the nature of the protective group, see "The Peptides, Analysis, Synthesis, Biology" Vol 9, Special Methods in Peptide Synthesis Part C, by J. A. Glaas, Enzymatic manipulation of Protecting Groups in Peptide Synthesis, Academic Press 1987.

The process of the invention may be performed with CPD-Y, which is the presently preferred enzyme, and which is characterised more fully in EP-B1-17485, and also with other serine or thiol carboxypeptidases, such as those mentioned in the survey below, since these have a common mechanism of activity via acyl enzyme intermediates.

| Enzyme | Origin |
|---|---|
| | Fungi |
| Penicillocarboxypeptidase S-1 | Penicillium janthinellum |
| - S-2 | - - |
| Carboxypeptidase(s) from | Aspergillus saitoi |
| - | Aspergillus oryzae |
| | Plants |
| Carboxypeptidase(s) C | Orange leaves |
| | Orange peels |
| Carboxypeptidase $C_N$ | Citrus natsudaidai |
| Hayata | |
| Phaseoline | Bean leaves |
| Carboxypeptidase(s) from | Sprouted barley or malt |
| | Sprouted cotton plants |
| | Tomatoes |
| | Watermelons |
| | Bromelein (pineapple) |
| | powder |

The close relation between many of the above-mentioned carboxypeptidases has been discussed by Kubota et al, Carboxypeptidase C, J. Biochem., Vol. 74, No. (1973), p. 757-770. Carboxypeptidases from malt, wheat and other sources have been described by Breddam, Carlsberg Res. Comm. Vol. 51, p. 83-128, 1986.

The carboxypeptidase used may also be chemically modified or be a biosynthetic mutant of the natural form.

As illustrated more fully below, the process of the invention is rather simple; however, it is important to maintain a resonably or substantially constant pH value in the reaction mixture. This pH value is between 5 and 10.5, and preferably between 7 and 9.5, but also depends upon the particular starting materials, the peptide formed and the enzyme.

The reaction is performed in an aqueous reaction medium, if desired containing up to 70% of an organic solvent which is miscible or immiscible with water, and compatible with the enzyme under the conditions specified. Preferred solvents are lower alcohols, dimethylformamide, dimethyl sulfoxide, dimethoxyethane, ethylene glycol and ethyl acetate.

The reaction temperature is preferably room temperature or above, e.g. 20 to 50 ° C, but temperatures in the range 0 to 60 ° C may be used. The most advantageous temperature may be chosen according to the

conditions otherwise given.

The concentration of the two reaction components may vary within wide limits, but the nucleophile component is frequently in excess. In order to avoid oligomerization of the substrate component, the nucleophile component is often added in small portions at intervals during the entire reaction sequence. If an ethyl or higher ester is used, surprisingly no oligomerization is observed, and very high substrate concentrations may be used without side-reactions. In this case, a homo-dipeptide in which A = B may be formed without oligomerization from a single starting component, the nucleophile being generated in situ by hydrolysis of this ester. In some cases, this is a further technical advantage, cf. Fig. 2.

Thus, the starting concentration of the substrate component may typically be 0.005 to 2 molar and for the nucleophile component, in cases where it is added separately, 0.005 to 3 molar. In most situations, it is possible to recover excess nucleophile component and the hydrolysis product from the substrate component for optional re-esterification and re-use. Recycling of the components is particularly easy because of their simple structure and the absence of side-reactions and deprotective losses.

The enzyme concentration may likewise vary, but is frequently somewhat higher (5-50 µm) than the concentrations appropriate for the use of N-protected amino-acid ester substrates. However, as illustrated by the following Examples, the amount required for synthetic purposes may be reduced more than ten-fold by using a stable immobilized enzyme preparation, thereby enabling the enzyme to be used in a continuous process.

The reaction medium may also contain salts, such as NaCl, which influence the binding of the enzyme to the substrate, as well as a complexing agent, such as EDTA, for metal ions present, which stabilises the enzyme.

The reaction rate differences between D- and L-substrates mentioned above are illustrated in Fig. 1, which shows the reaction sequence for the CPD-Y hydrolysis of a protected and unprotected amino-acid ester (Tyr-OEt) in D- and L-form. It will be seen from the Figure that, while L-AcTyrOEt is hydrolyzed almost instantaneously, only insignificant hydrolysis (below 5%) of D-AcTyrOEt has taken place after 2 hours.

In contrast, thre are only small differences in the hydrolysis sequence for the unprotected esters in L- and D-form.

This surprising hydrolysis sequence is reflected in the following Examples, which illustrate the production of various dipeptides in the process of the invention using the enzymes CPD-Y and malt carboxypeptidase II, (CPD-MII) and wheat carboxypeptidase (CPA-W).

General method for Examples 1-15

The reactions, performed on an analytical scale with a reaction volume of 1 ml, were carried out in a pH-stat, and the selected pH value was kept constant by automatic addition of 1 N NaOH. Reaction temperature was room temperature, unless otherwise stated. The table also includes reaction concentrations, content of organic solvent, product and yield. Reaction times are typically between 0.5 and 5 hours, and enzyme concentrations are typically 10-20 µm, unless otherwise stated.

Product identification and determination of product yield were performed by means of reverse phase HPLC (Waters 6000 A pumps, 660 gradient blender, UK 6 injector) on a $C_{18}$ NOVA PAK column (Waters, RCM) using suitable gradients of elution systems containing 50 mM triethylammonium phosphate, pH 3.0 from 0% to 80% acetonitrile with a flow of 2 ml/min. Elution was monitored by means of a UV detector (Waters 480) at 230 nm, 254 nm, 278 nm or 290 nm.

The products were identified by amino-acid analysis of fractions from the HPLC analysis, which corresponded to the assumed product peak and/or by HPLC comparison with a chemically-synthesized reference product. These were produced according to known principles, usually via reaction between BOC-A-OSu - tertiary-butyloxycarbonyl - the succine imide ester derivative of the substrate amino-acid - and the used nucleophile component followed by deblocking of the N-terminal amino acid residue. In all cases, it was possible to separate LL-and DD-dipeptides from the diasteromeric DL-and LD-dipeptide products.

For the products which can only be detected at 230 nm, the product yields were determined by means of the absorption/concentration curve of the chemically synthesized reference compound. For the other products, the yields were determined on the basis of the ratio between the integrated areas below the peaks in the elution chromatogram, corresponding to product respectively the reactant which absorbs at the wavelength concerned.

The reaction conditions in the preparative Examples 16-21 are described in the individual Examples. The reactions were followed on analytical HPLC as described. The enzyme concentrations are generally lower and the reaction times longer than in the corresponding analytical Examples, but no attempt to optimize the reaction conditions has been made.

Example 1

| Carboxypeptidase Y [a]) catalyzed synthesis of L-L-dipeptides with L-Tyrosine ethyl ester (50 mM) as substrate component and free amino-acids as nucleophiles | | | | | |
|---|---|---|---|---|---|
| Nucleophile | (conc.) | Solvent | pH | Product | Yield % |
| Alanine | (1.9 M) | Water | 9.5 | TyrAlaOH | 10 |
| Arginine | (0.8 M) | Water | 9.5 | TyrArgOH | 31 |
| Cysteine | ( 1 M ) | Water | 8.0 | TyrCysOH | 30 |
| LD-Cysteine | ( 2 M ) | Water | 8.0 | TyrCysOH(LL) | 40 |
| Leucine | (0.2 M) | Water | 8.0 | TyrLeuOH | 1 |
| Lysine | ( 2 M ) | Water | 9.5 | TyrLysOH | 18 |
| Methionine | (0.3 M) | Water | 8.0 | TyrMetOH | 8 |
| Methionine | (0.3 M) | Water | 9.0 | TyrMetOH | 9 |
| Methionine | (0.3 M) | 30% DMSO | 9.0 | TyrMetOH | 15 |
| Methionine | (0.3 M) | 15% EtOH | 9.0 | TyrMetOH | 7 |
| Glutamine | (0.8 M) | Water | 9.5 | TyrGlnOH | 8 |
| Pennicilamine | (0.5 M) | Water | 8.0 | TyrPenOH | 7 |

[a]) 10 µM, 1 mM EDTA

Example 2

| Carboxypeptidase Y [a]) catalyzed synthesis of L-L-dipeptides with L-Methionine (0.3 M) as nucleophile component in water at pH 9.0 | | |
|---|---|---|
| Substrate (50 mM) | Product | Yield % |
| Leucine methyl ester | LeuMetOH | 30 |
| Leucine isopropyl ester | LeuMetOH | 36 |
| Methionine ethyl ester[b]) | MetMetOH | 25 |
| Phenylalanine methyl ester | PheMetOH | 16 |
| Phenylalanine ethyl ester | PheMetOH | 19 |
| Phenylalanine isopropyl ester | PheMetOH | 23 |
| Serine isopropyl ester[c]) | SerMetOH | 21 |
| Tryptophane methyl ester | TrpMetOH | 26 |
| Tyrosine benzyl ester[d]) | TyrMetOH | 19 |

[a]) 10 µM. 1 mM EDTA
[b]) 5 mM
[c]) Reaction time > 20 hr
[d]) 30% DMSO

Example 3

| Carboxypeptidase Y [a]) catalyzed synthesis of L-L-dipeptides with L-Tyrosine ethyl ester (50 mM) as substrate component and L-amino-acid amides or esters as nucleophiles | | | | | | |
|---|---|---|---|---|---|---|
| Substrate | Nucleophile | (Conc.) | Solvent | pH | Product | Yield% |
| TyrOEt | Leucine amide | (0.2 M) | 30% DMSO | 9.5 | $TyrLeuNH_2$ [b]) | 42 |
| | | | | | TyrLeuOH | 4 |
| TyrOEt | Lysine amide | (0.3 M) | Water | 9.5 | $TyrLysNH_2$ | 20 |
| | | | | | TyrLysOH | 22 |
| TyrOEt | Arginine amide | (0.2 M) | Water | 9.0 | $TyrArgNH_2$ | 50 |
| TyrOEt | Valine amide | (0.3M) | Water | 9.0 | $TyrValNH_2$ | 77 |
| TyrOEt | Leucine methyl ester | (0.2 M) | 30% DMSO | 9.0 | TyrLeuOH | 6 |

[a]) 20 µM, 1 mM EDTA
[b]) Reaction time > 20 hr, 50% substrate converted

Example 4

| Carboxypeptidase Y [a]) catalyzed synthesis of L-L-homodipeptides from a single starting compound in water at pH 8.5 | | | |
|---|---|---|---|
| Substrate | (conc.) | Product | Yield % |
| Methionine methyl ester | (0.5 M)[b])[c]) | MetMetOH | 14 |
| Methionine ethyl ester | (0.5 M) | MetMetOH | 16 |
| Methionine isopropyl ester | (0.5 M) | MetMetOH | 14 |
| Tyrosine methyl ester | (0.2 M) | TyrTyrOH [c]) | 1 |
| Tyrosine ethyl ester | (0.2 M) | TyrTyrOH [c]) | 1 |
| Phenylalanine ethyl ester | (0.2 M)[d])[e]) | PhePheOH | 2 |
| Alanine amide | (0.2 M)[f])[d]) | $AlaAlaNH_2$ | [b]) |

[a]) 10 µM CPD-Y, 1 mM EDTA
[b]) Polymerization
[c]) Precipitation
[d]) pH 9.0
[e]) 30% DMSO
[f]) 50 uM CPD-Y, 1 mM EDTA

Example 5

| Carboxypeptidase Y [a]) catalyzed synthesis of D,L-dipeptides with D-Tyrosine ethyl ester (50 mM) as substrate and free L-amino-acids as nucleophiles in water | | | | |
|---|---|---|---|---|
| Nucleophile | (conc.) | pH | Product | Yield % |
| Arginine | ( 1 M ) | 9.0 | tyrArgOH | 75 |
| Cysteine | ( 1 M ) | 8.0 | tyrCysOH | 86 |
| LD-cysteine | ( 2 M ) | 8.0 | tyrCysOH(DL) | 45 |
| Leucine | (0.2 M) | 8.0 | tyrLeuOH | 22 |
| Methionine | ( 1 M ) | 9.0 | tyrMetOH | 65 |
| Pennicilamine | ( 1 M )[b]) | 8.0 | tyrPenOH | 27 |

[a]) 10 µM, 1 mM EDTA
[b]) Reaction time > 20 hr

Example 6

| Carboxypeptidase Y [a]) catalyzed synthesis of D,L-dipeptides with L-Methionine (0.3 M) as nucleophile component in water at pH 9.0 | | |
|---|---|---|
| D-substrate (50 mM) | Product | Yield % |
| leucine methyl ester | leuMetOH | 50 |
| leucine isopropyl ester | leuMetOH | 71 |
| methionine ethyl ester | metMetOH | 68 |
| phenylalanine ethyl ester | pheMetOH | 71 |
| phenylalanine isopropyl ester | pheMetOH | 72 |
| serine isopropyl ester | serMetOH [b]) | 46 |
| tryptophane ethyl ester | trpMetOH | 72 |

[a]) 15 µM, 1 mM EDTA
[b]) Reaction time 5 days

Example 7

| Carboxypeptidase Y [a]) catalyzed synthesis of D,L-dipeptide amides with D-Tyrosine or D-Phenylalanine ethylester (50 mM) as substrate component and L-amino-acid amides as nucleophile components in water | | | | | |
|---|---|---|---|---|---|
| Substrate | Nucleophile | (conc.) | pH | Product | Yield % |
| tyrOEt | Leucine amide | (0.2 M) | 9.0 | $tyrLeuNH_2$ | 82 |
| | | | | tyrLeuOH | 2 |
| tyrOEt | Valine amide | (0.3 M) | 9.0 | $tyrValNH_2$ | 94 |
| tyrOEt | Arginine amide | (0.2 M) | 9.0 | $tyrArgNH_2$ | 86 |
| pheOEt | Alanine amide | (0.8 M) | 9.0 | $pheAlaNH_2$ [b]) | 50 |

[a]) 15 µM, 1 mM EDTA
[b]) Some polymerization noted

Example 8

| Carboxypeptidase Y [a]) catalyzed synthesis of D,D-homodipeptide esters from D-substrate ester components in water at pH 9.0, also acting as nucleophile component | | | |
|---|---|---|---|
| D-substrate | (conc.) | Product | Yield % |
| tyrosin ethyl ester | (0.05 M) | tyrtyrOEt | 9 |
| phenylalanine ethyl ester | (0.1 M) | phepheOEt | 10 |
| tyrosine ethylene glycol ester | (0.05 M) | tyrtyrOEtOH | 1 |
| methionine methyl ester | (0.1 M) | metmetOMe | 3 |

[a]) 15 µM, 1 mM EDTA

Example 9

| Carboxypeptidase Y [a]) catalyzed synthesis of L-L-dipeptides with side-chain-protected carboxy-terminal amino-acids with L-TyrOEt (50 mM) as substrate component and side-chain-protected L-amino-acids and amides as Nucleophiles | | | | | |
|---|---|---|---|---|---|
| Nucleophile | (Conc.) | pH | Solvent | Product | Yield% |
| Acetamidomethyl cysteine | (1 M) | 8.5 | Water | TyrCys(-SAcm)OH | 10 |
| Acetamidomethyl cysteine amide | (0.4 M) | 8.5 | Water | $TyrCys(-SAcm)NH_2$<br>TyrCys(-SAcm)OH | 12<br>1 |
| Beta-Benzylaspartic Acid<br>Epsilon Trifluoroacetyllysine | (0.1 M)[b]<br>(0.1 M)[b] | 9.0<br>8.5 | 30%DMSO<br>30%DMSO | TyrAsp(OBzl)OH<br>TyrLys(Tfa)OH | 13<br>12 |
| Gamma-Tert-butylglutamic Acid Amide | (0.1 M)[b] | 8.0 | 30%DMSO | $TyrGlu(OtBu)NH_2$<br>TyrGlu(OtBu)OH | 3<br>12 |
| Gamma-Methylglutamic Acid<br>Gamma-Ethylglutamic Acid | (0.3 M)<br>(0.3 M) | 8.5<br>8.5 | Water<br>Water | TyrGlu(OMe)OH<br>TyrGlu(OEt)OH | 20<br>18 |

[a]) 10 µM, 1 mM EDTA
[b]) 25 mM Substrate, 20 µm

Example 10

| Carboxypeptidase Y [a]) catalyzed synthesis of D-L-dipeptides with side-chain-protected carboxy-terminal amino-acids with D-TyrOEt (50 mM) as substrate component and side-chain-protected L-amino-acids and amides as Nucleophiles | | | | | |
|---|---|---|---|---|---|
| Nucleophile | (Conc.) | pH | Solvent | Product | Yield% |
| Acetamidomethylcysteine | (1 M) | 8.5 | Water | tyrCys(-SAcm)OH | 75 |
| Acetamidomethylcysteine amide | (0.4 M) | 8.5 | Water | tyrCys(-SAcm)$NH_2$ | 71 |
| | | | | tyrCys(-SAcm)OH | 6 |
| Beta-Benzylaspartic Acid | (0.1 M)[b] | 9.0 | 30%DMSO | tyrAsp(OBzl)OH | 54 |
| Epsilon-Trifluoroacetyllysine | (0.1 M)[b] | 8.5 | 30%DMSO | tyrLys(Tfa)OH | 51 |
| Gamma-Tert-butylglutamic Acid Amide | (0.1 M)[b] | 8.0 | 30%DMSO | tyrGlu(OtBu)$NH_2$ | 42 |
| | | | | tyrGlu(OtBu)OH | 10 |
| Gamma-Methylglutamic Acid | (0.3 M) | 8.5 | Water | tyrGlu(OMe)OH | 75 |
| Gamma-Ethylglutamic Acid | (0.3 M) | 8.5 | Water | tyrGlu(OEt)OH | 71 |

[a]) 10 µM, 1 mM EDTA
[b]) 25 mM Substrate, 20 µm

Example 11

| Carboxypeptidase Y [a]) catalyzed synthesis of L,L-dipeptides with side-chain-protected amino-terminal amino-acid residues from side-chain-protected substrate components (25 mM) and L-Methionine (0.3 M) as nucleophile component in 30% DMSO at pH 9.0 | | |
|---|---|---|
| Substrate | Product | Yield % |
| L-Aspartic Acid Dibenzyl ester [b]) | Asp(OBzl)MetOH | 65 |
| L-Glutamic Acid Dibenzyl ester [c]) | Glu(OBzl)MetOH | 70 |

[a]) 20 µM, 1 mM EDTA, Reaction time > 20 h
[b]) At 35 per cent conversion
[c]) At 60 per cent conversion

Example 12

| Carboxypeptidase Y [a]) catalyzed synthesis of dipeptide esters and amides containing omega-amino-acids from L- and D-amino-acid ester substrates at 50 mM in water with beta-alanine and beta-alanine amide as nucleophiles | | | | | |
|---|---|---|---|---|---|
| Substrate | Nucleophile | (conc.) | pH | Product | Yield % |
| tyrOEt | B-Alanine methyl ester | (0.2 M) | 8.5 | tyrBAlaOMe | 51 |
| tyrOEt | B-Alanine methyl ester | (0.5 M) | 8.5 | tyrBAlaOMe | 72 |
| pheOEt | B-Alanine methyl ester | (0.5 M) | 9.0 | pheBAlaOMe | 83 |
| PheOEt | B-Alanine methyl ester | (0.5 M) | 9.0 | PheBAlaOMe | 15 |
| pheOEt | B-Alanine amide | (0.5 M) | 9.0 | pheBAla$NH_2$ | 70 |
| PheOEt | B-Alanine amide | (0.5 M) | 9.0 | PheBAla$NH_2$ | 3 |

[a]) 20 μM, 1 mM EDTA

Example 13

Carboxypeptidase Y [a]) catalyzed synthesis of L,L and D,L dipeptides with hydroxyalkyl esters of L- and D-Tyrosine and L-Phenylalanine (50 mM) as substrate components and free L-Methionine (0.3 M) as nucleophile in water/ethylene glycol mixtures at pH 9.0

| Substrate | % glycol (vol/vol) | Product | Yield % |
|---|---|---|---|
| TyrOEtOH | 0 | TyrMetOH | 10 |
| TyrOEtOH | 40 | TyrMetOH | 8 |
| TyrOEtOH | 60[b]) | TyrMetOH | 5 |
| tyrOEtOH | 0 | tyrMetOH | 56 |
| PheOEtOH | 0 | PheMetOH | 16 |

[a]) 5 $\mu$M, 1 mM EDTA
[b]) 10 $\mu$M, 1 mM EDTA

Example 14

| Synthesis of L,L-dipeptides catalyzed by carboxypeptidases from barley and wheat at 50 mM initial L-substrate ester concentration at pH 8.0 in water and L-amino-acid and amides as nucleophile components | | | | | |
|---|---|---|---|---|---|
| Enzyme | Substrate | Nucleophile | (conc.) | Product | Yield% |
| CPD-MII[a]) | PheOEt | Methionine | (0.4 M) | PheMetOH | 10 |
| CPD-W[b]) | PheOEt | Arginine | (0.8 M) | PheArgOH | 8 |

[a]) 20 μM, 1 mM EDTA, 2 M NaCl
[b]) 10 μM, 1 mM EDTA

Example 15

| Synthesis of D,L-dipeptides catalyzed by carboxypeptidases from barley and wheat at 50 mM initial D-Phenylalanine ethyl ester concentration at pH 8.0 in water and free L-amino-acids as nucleophile components | | | | | |
|---|---|---|---|---|---|
| Enzyme | Substrate | Nucleophile | (conc.) | Product | Yield% |
| CPD-MII[a]) | pheOEt | Methionine | (0.4 M) | pheMetOH | 15 |
| CPD-W[b]) | pheOEt | Arginine | (0.8 M) | pheArgOH | 13 |
| CPD-W[b]) | pheOEt | Methionine | (0.4 M) | pheMetOH | 40 |

[a]) 20 μM, 1 mM EDTA, 2 M NaCl
[b]) 10 μM, 1 mM EDTA, 2 M NaCl, Reaction time > 20 hr, conversion less than 50 per cent

Example 16

Preparative synthesis of L,L-tyrosylcysteine, TyrCysOH

Procedure

L-tyrosine ethyl ester hydrochloride (1.5 g, 6 mmol) and L-cysteine (15.2 g, 125 mmol) were dissolved in 110 ml of-0,1 M KCl solution containing 1 mM EDTA. pH was adjusted to 8.0 with triethylamine. The reaction was initiated by addition of 7.5 ml of carboxypeptidase Y-solution (16 mg/ml), and was kept at pH 8.0 for the duration of the reaction by the addition of triethylamine under continuous stirring at room temperature. The remainder of the substrate (13.5 g, 54 mmol) was added in portions of 1,5 g during one hour. After 0,5 hour tyrosine precipitation started, and after 3.5 hours the reaction was stopped by heating to 45 °C for 20 minutes.

The formed tyrosine was filtered off, and the filtrate was purified by R-preparative HPLC (Waters Prep. L C/system 500A) using two columns (5.7 x 30 cm) packed with 60 um $C_{18}$-particles and 50 mM acetic acid as eluent. Collected fractions containing pure product were evaporated to dryness in vacuo under repeated additions of absolute ethanol. The remnant was stirred with diethyl ether. Following this, 3.88 g of L,L-tyrosine cysteine (14 mmol, 22%) was isolated by filtration and drying.

Identification

Choride and acetate were not detected, the product being present as a zwitter-ion.

Amino-acid analysis following acid hydrolysis and derivation of Cys by acrylonitrile gave the result:

Tyr (1.00)

Cys (1.08)

Purity

Following derivation of the cysteine sidechain with acrylonitrile, only one spot was detected on TLC on Kiselgel 60-F using ninhydrine detection (Rf 0.73, eluent : ethyl acetate, butanol, acetic acid and water (1:1:1:1)).

HPLC-purity: 99.5% (nucleosile 7 $C_{18}$, 0.1 M ammonium phosphate, pH 3.0/acetonitrile, 220 nm).

UV-quantization: 98.5% (293 nm, tyrosine absorbance in 0,1 M NaOH).

Example 17

Preparative synthesis of L,L-methionyl-methionine, MetMet-OH

Procedure

L-methionine ethyl ester hydrochloride (24.6 g, 115 mmol) and L-methionine (20.6 g, 138 mmol) were dissolved in 190 ml of 0.1 M KCl solution containing 1 mM EDTA. pH was adjusted to 9.0 with sodium hydroxide solution, and the reaction was initiated by addition of 14.2 ml of carboxypeptidase Y solution (20 mg/ml). The reaction was stirred overnight at room temperature, and pH was kept at 9.0 through the addition of sodium hydroxide solution by a pH-stat. pH was adjusted to 3.0 with HCl-solution at the end of reaction.

Precipitated methionine was filtered off, and the filtrate was purified by R-preparative HPLC as described in example 16. Collected fractions containing pure product were concentrated by evaporation and finally freeze-dried. This procedure gave 10.6 g (37.8 mmol, 33%) of L-methionyl-L-methionine as a white amorphous powder.

Indentification

No chloride was detected, and only 1.9% (w/w) of acetate was determined, the product being predominantly in zwitter-ionic form. Amino-acid analysis showed methionine following acid hydrolysis and the absence of free methionine in unhydrolysed samples.

Purity

Purity by HPLC: 99.8% (nucleosile 7 $C_{18}$, 0.1 M ammonium phosphate, pH 3.0/acetonitrile, 220 nm).

Quantization by reaction with trinitrobenzenesulfonic acid (TNBS) and UV-detection: 92.5%.

Water content by Karl Fisher: 1.5%.

Example 18

Preparative synthesis of L,L-tyrosylvalineamide, $TyrValNH_2$

Procedure

L-tyrosine ethyl ester hydrochloride (16.0 g, 65 mmol) and L-valine amide hydrochloride (60 g, 390 mmol) were dissolved in 1150 ml of water and 65 ml of 20 mM EDTA was added. pH was adjusted to 9.0 with sodium hydroxide solution and the reaction was initiated by addition of 12 ml of a carboxypeptidase Y solution (20 mg/ml). The reaction was stirred at room temperature for four hours, and pH was maintained at 9.0 by the addition of sodium hydroxide solution. Following complete conversion the reaction was stopped by raising pH to 11.

The denatured enzyme was filtered off, and the reaction mixture was diluted and purified by successive anion exchange on a Dowex A61x4 column and cation exchange on a Dowex A650Wx4 column using soidum and ammonium acetate salt gradients, respectively, and was finally desalted. Product fractions were combined and evaporated to dryness in vacuo under repeated additions of absolute ethanol, giving 11.2 g L,L-tyrosylvaline amide (40 mmol, 62%) as a white powder.

Identification

1.8% (w/w) of acetate was determined, the product being predominantly in zwitter-ionic form.
Amino-acid analysis following acid hydrolysis gave the following results:
Tyr (1.01)
Val (0.99)

Purity

HPLC-purity: 99.5% (Novapak $C_{18}$, 0.1 M ammonium phosphate containing alkylsulfonate, pH 4.5/acetonitrile, 220 nm).
UV-quantization: 97.8% (293 nm, tyrosine absorbance in 0.1 M NaOH).

Example 19

Preparative synthesis of D,L-tyrosyl-arginine, D,L-tyrArg-OH

Procedure

105 g of L-arginine (105 g, 603 mmol) was dissolved in 400 ml of water, and pH was adjusted to 9.0 with HCl-solution. D-tyrosine ethyl ester hydrochloride (6.1 g, 25 mmol) and 5 ml of 0.1 M EDTA were added, and pH readjusted to 9.0. The reaction was initiated by addition of 7 ml of a carboxypeptidase Y solution (20 mg/ml) and pH was kept at 9.0 by addition of NaOH-solution. After 4 hours the reaction was stopped by adjusting pH to 3 with HCl-solution.

The reaction mixture was diluted and purified by cation exchange on a Dowex A650Wx4 column using an ammonium acetate salt gradient, and finally desalted. Collected product fractions were concentrated by evaporation and finally freeze-dried, giving 6.45 g of D,L-tyrosyl-arginine (19 mmol, 77%) as a white powder.

Identification

Acetate and chloride could not be detected, the product being present as a zwitter-ion.
Amino-acid analysis following acid hydrolysis gave the following result:
Arg (1.03)
Tyr (0.97)

Purity

HPLC-purity: 99.5% (Novapak $C_{18}$, 0.1 M ammonium phosphate with alkylsulfonic acid, pH 4.5/acetonitrile, 220 nm).
Water content by Karl Fisher: 3.4%.

Example 20

Preparative synthesis of D,L-phenylalanylmethionine, D,L-pheMetOH by the use of immobilized carboxypeptidase Y

Immobilization procedure

Carboxypeptidase Y was immobilized on Eupergit C following the recommended procedure of the manufacturer. The immobilization was carried out in the phosphate buffer at pH 7.5, and residual active gel-groups were blocked with ethanolamine at pH 8.0 with subsequent washing.

93% of the enzyme was bound to the gel containing 2.5 mg protein/ml. The Eupergit C coupled enzyme was stored in 10 mM PIPES, 1 mM EDTA, 0.05% hydroxybenzoic acid ethyl ester, pH 7.0 at 4°C.

Synthetic procedure

D-phenylalanine ethyl ester hydrochloride (5.7 g, 25 mmol) and L-methionine (29.8, 200 mmol) were dissolved in 400 ml of $H_2O$, 5 ml of 0.1 N EDTA was added, and pH adjusted to 9.0 with sodium hydroxide solution, giving a reaction volume of 500 ml. The reaction mixture was continuously stirred and pH kept constant at 9.0 with sodium hydroxide solution, while the solution was being circulated over a column of immobilized CPD-Y at a flowrate of 3 ml/min. The coloumn contained immobilized CPD-Y on Eupergit C prepared as described above and was 2.5 cm x 5.5 cm with a volume of 27 ml, containing a total of 67 mg of CPD-Y. Circulation was continued for 10 hours. pH was then adjusted to 7 with HCl-solution and the product was purified on R-preparative HPLC as described in Example 16.

Collected fractions containing pure product were combined, evaporated in vacuo and finally freeze-dried to give 3.5 g (12 mmol, 48%) of D,L-phenylalanylMethionine as a white, armorphous powder.

Stability of the enzyme preparation

The experiment could be repeated with the same enzyme gel preparation several times without noticable loss in conversion rate and comparable results. Thus, the enzyme is fairly stable at the reaction conditions, further improving process economy.

Indentity of the product

The product was free of chloride, but contained 7.0% (w/w) of acetate and so was partially on acetate form.

Amino-acid analysis showed the absence of free amino-acids, and following acid hydrolysis a ratio of

Met (0.98)

Phe (1.03)

Specific optical rotation using sodium D-line at 25°C and c = 0.25 in water was -128.9°

Purity of product

HPLC: 99.6% (nucleosile 7 $C_{18}$, 0.1 M ammonium phosphate, pH 3/acetonitrile, 220 nm).

Water content by Karl Fisher: 2.8%

Example 21

Preparative synthesis of D,D-phenylalanylphenylalanine ethyl ester hydrochloride, D,D-phepheOEt.HCl

Procedure

D-phenylalanine ethyl ester hydrochloride (2.5 g, 11 mmol) was dissolved in 45 ml of water, and 0.5 ml of 0.1 N EDTA was added. pH was adjusted to 9.0 with sodium hydroxide solution, the substrate being present as a partially oily suspension at the beginning. The reaction was initiated by addition of 3.4 ml of a carboxypeptidase Y solution (20 mg/ml) and was stirred for 2.5 hours at room temperature, pH being kept at 9.0 with sodium hydroxide solution. The reaction was stopped by adjusting pH to 3 with HCl-solution.

The reaction mixture was filtered and purified by R-preparative HPLC as described in example 16.

Collected product fractions were concentrated by evaporation in vacuo and freeze-dried with added HCl-solution, giving 0.49 g (1.3 mmol, 12%) as a white, amorphous powder.

Identification

The product contained 9.2% (w/w) of chloride and no acetate, the product being present as hydrochloride.

Amino-acid analysis showed phenylalanine following acid hydrolysis and no phenylalanine prior to acid hydrolysis.

The product could be further hydrolysed with base, yielding a product chromatographically different from D,L-Phe-PheOH, and the product itself co-eluted with an L,L-PhePhe-OEt-standard in the used HPLC-system.

Finally, specific optical rotation using sodium D-line at 25°C and c=0.5 in acetic acid was found to be -42.7°. This compared with a pure standard of L,L-PhePheOEt, which gave +52.1° under similar conditions. In this case, the discrepancy is believed to be due to the lesser purity of the synthesized peptide.

Purity

HPLC-purity: 82.3% (nucleosile 7 $C_{18}$, 0.1 M ammonium phosphate, pH 3/acetonitrile, 220 nm).

The impurities detected were chromatographically different from substrate, substrate hydrolysis, product hydrolysis or diastereomers thereof.

Water content by Karl Fisher: 7.5%.

**Claims**

**1.** A process for producing dipeptides having the general formula

H-A-B-Y

wherein A represents an optionally side-chain-protected L- or D-α-amino-acid residue or ω-amino-acid residue, and B represents an optionally side-chain-protected L- or D-α-amino-carboxylic acid residue which may be the same as or different from A, an L- or D-aminophosphonic acid residue or an L- or D-aminosulfonic acid residue or the corresponding ω-amino-acid or a salt or hydrate thereof, and Y is OH or a C-terminal blocking group, **characterized** by reacting a substrate component, which is an amino-acid derivative having the formula

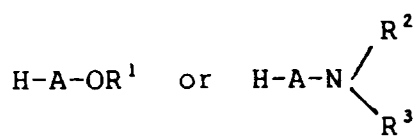

wherein A is as defined above, $R^1$ represents hydrogen, alkyl, aryl or aralkyl optionally substituted with inert substituents or an α-des-amino fragment of an amino-acid, and $R^2$ and $R^3$ are the same or different and each represent hydrogen, alkyl, aryl or aralkyl optionally substituted with inert substituents, with a nucleophile component (which, when A = B may be formed in situ) selected from

(a) amino-acids having the formula

H-B-OH

wherein B is an aminocarboxylic acid residue as defined above,

(b) amino-acid amides having the formula

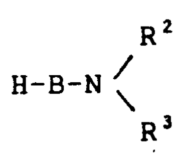

wherein B is an aminocarboxylic acid residue as defined above, and $R^2$ and $R^3$ have the above meaning, except that when $R^2$ represents hydrogen, $R^3$ may also represent hydroxy or amino,

(c) L-amino-acid esters having the formula

H-B-$OR^4$

wherein B is an aminocarboxylic acid residue as defined above, and $R^4$ represents alkyl, aryl or aralkyl, and

(d) straight-chain or branched aminophosphonic acids or aminosulfonic acids having the formula

$NH_2C_xH_zPO_3H_2$ or $NH_2C_xH_zSO_3H$

wherein x is 1-6 and z is 2-12,

in the presence of a serine or thiol carboxypeptidase from yeast or of animal, vegetable or other microbial origin, in an aqueous solution or suspension having a pH value between 5 and 10.5 and optionally containing an organic solvent and/or a salt, and then, if desired, cleaving any side-chain-protecting group or protective group Y and/or, if desired, converting the resulting dipeptide derivative to a salt or hydrate.

**2.** A process according to claim 1, **characterized** by using carboxypeptidase Y from yeast as the carboxypeptidase.

**3.** A process according to claim 2, **characterized** in that the carboxypeptidase used has been purified by affinity chromatography over an affinity resin consisting of a polymeric resin skeleton with a plurality of coupled benzyl succinyl groups.

**4.** A process according to claim 1, **characterized** in that the carboxypeptidase used is penicillocarboxypeptidase S-1 or S-2 from Penicillium janthinellum, a carboxypeptidase from Aspergillus saitoi or Aspergillus oryzae, a carboxypeptidase C from orange leaves or orange peels, carboxypeptidase $C_N$ from Citrus natsudaidai Hayata, phaseoline from bean leaves or a carboxypeptidase from sprouted barley, malt, sprouted cotton plants, tomatoes, watermelons or Bromelein (pineapple) powder.

**5.** A process according to claims 1-4, **characterized** in that the carboxypeptidase used has been chemically modified or is a biosynthetic mutant of a natural form.

**6.** A process according to any of the preceding claims, **characterized** in that the carboxypeptidase enzyme used has been immobilized.

**7.** A process according to any of the preceding claims, **characterized** by using an aqueous reaction solution or reaction dispersion containing from 0 to 70% of an organic solvent.

**8.** A process according to claim 7, **characterized** in that the organic solvent is selected from alkanols, dimethyl sulfoxide, dimethylformamide, dimethoxyethane, ethylene glycol or ethyl acetate.

**9.** A process according to any of the preceding claims, **characterized** by using as substrate component a D- or L-amino-acid ester selected from benzyl esters or straight or branched $C_1$-$C_6$ alkyl esters optionally substituted with inert substituents.

**10.** A process according to any of the preceding claims, **characterized** by using as nucleophile component an amino-acid or amino-acid amide having the formula

H-B-OH or H-B-$NHR^3$

wherein $R^3$ is hydrogen or $C_1$-$C_3$ alkyl and B is an L-amino-acid residue.

**11.** A process according to claim 1, **characterized** by using as nucleophile component an ester having the formula

H-B-$OR^4$

wherein B is an aminocarboxylic acid residue and $R^4$ is $C_1$-$C_3$ alkyl.

**12.** A process according to any of the preceding claims, **characterized** in that the resulting dipeptide includes one or more C-terminal protective groups Y, and that the group or groups are cleaved enzymatically, preferably by means of the same carboxypeptidase enzyme as was used in the preceding reaction.

13. A process according to any of the preceding claims, **characterized** in that the resulting dipeptide includes one or more side-chain protective groups and that the group or groups are cleaved enzymatically, preferably by means of an esterase or lipase or proteolytic enzyme.

**Patentansprüche**

1. Verfahren zur Herstellung von Dipeptiden der allgemeinen Formel

H-A-B-Y

in der
A einen gegebenenfalls seitenkettengeschützten L- oder D-alpha-Aminosäurerest oder omega-Aminosäurerest und B einen gegebenenfalls seitenkettengeschützten L- oder D-alpha-Aminocarbonsäurerest, der gleich oder verschieden von A ist, einen L- oder D-Aminophosphonsäurerest oder einen L- oder D-Aminosulfonsäurerest oder die entsprechende omega-Aminosäure oder ein Salz oder ein Hydrat derselben bedeuten und Y OH oder eine C-terminale Blockierungsgruppe ist, dadurch gekennzeichnet, daß man eine Substratkomponente, die ein Aminosäurederivat der Formel

H-A-$OR^1$ oder H-A-$NR^2R^3$

in der A wie oben definiert ist, sowie $R^1$ Wasserstoff, Alkyl, Aryl oder Aralkyl, gegebenenfalls substituiert mit inerten Substituenten, oder ein alpha-Desaminofragment einer Aminosäure bedeutet und $R^2$ und $R^3$ gleich oder verschieden sind und jeweils Wasserstoff, Alkyl, Aryl, Aralkyl, gegebenenfalls substituiert mit inerten Substituenten, bedeuten, mit einer nucleophilen Komponente (die, wenn A = B ist, in situ gebildet werden kann), ausgewählt aus
(a) Aminosäuren der Formel

H-B-OH

in der B ein wie oben definierter Aminocarbonsäurerest bedeutet,
(b) Aminosäureamiden der Formel

H-B-$NR^2R^3$

in der B ein wie oben definierter Aminocarbonsäurerest sowie $R^2$ und $R^3$ wie oben definiert sind, ausgenommen daß, wenn $R^2$ Wasserstoff ist, $R^3$ auch Hydroxy oder Amino sein kann,
(c) L-Aminosäureestern der Formel

H-B-$OR^4$

in der B ein wie oben definierter Aminocarbonsäurerest ist und R Alkyl, Aryl oder Aralkyl bedeutet, und
(d) geradkettigen oder verzweigten Aminophosphonsäuren oder Aminosulfonsäuren der Formeln

$NH_2C_xH_zPO_3H_2$ oder $NH_2C_xH_zSO_3H$

in der x 1-6 und z 2-12 sind,
in Gegenwart einer Serin- oder Thiolcarboxypeptidase aus Hefe oder tierischen, pflanzlichen oder anderen mikrobiellen Ursprungs in einer wässrigen Lösung oder Suspension mit einem pH-Wert zwischen 5 und 10,5, die gegebenenfalls ein organisches Lösemittel und/oder ein Salz enthält, umsetzt, und anschließend, falls erwünscht, jegliche Seitenkettenschutzgruppen oder Schutzgruppen Y abspaltet und/oder, falls erwünscht, das erhaltene Dipeptidderivat in ein Salz oder Hydrat überführt.

2. Verfahren nach Anspruch 1, dadurch gekennzeichnet, daß man Carboxypeptidase Y aus Hefe als Carboxypeptidase verwendet.

3. Verfahren nach Anspruch 2, dadurch gekennzeichnet, daß die verwendete Carboxypeptidase mittels Affinitätschromatographie über einem Affinitätsharz gereinigt worden ist, welches aus einem polymeren

Harzskelett mit einer Vielzahl von angekuppelten Benzylsuccinylgruppen besteht.

4. Verfahren nach Anspruch 1, dadurch gekennzeichnet, daß die verwendete Carboxypeptidase Penicillocarboxypeptidase S-1 oder S-2 aus Penicillum janthinellum, eine Carboxypeptidase aus Aspergillus saitoi oder Aspergillus oryzae, eine Carboxypeptidase C aus Orangenblättern oder Orangenschalen, Carboxypeptidase $C_N$ aus Citrus natsudaidai Hayata, Phaseolin aus Bohnenblättern oder eine Carboxypeptidase aus gekeimter Gerste, Malz, gekeimten Baumwollpflanzen, Tomaten, Wassermelonen oder Bromelein-(Ananas)-Pulver ist.

5. Verfahren gemäß den Ansprüchen 1 bis 4, dadurch gekennzeichnet, daß die eingesetzte Carboxypeptidase chemisch modifiziert oder ein biosynthetischer Mutant einer natürlichen Form ist.

6. Verfahren gemäß einem jeden der obigen Ansprüche, dadurch gekennzeichnet, daß das verwendete Carboxypeptidaseenzym immobilisiert ist.

7. Verfahren gemäß einem jeden der obigen Ansprüche, dadurch gekennzeichnet, daß man eine wässrige Reaktionslösung oder Reaktionsdispersion verwendet, die von 0 bis 70% eines organischen Lösemittels enthält.

8. Verfahren nach Anspruch 7, dadurch gekennzeichnet, daß das organische Lösemittel aus Alkanolen, Dimethylsulfoxid, Dimethylformamid, Dimethoxyethan, Ethylenglykol oder Ethylacetat ausgewählt ist.

9. Verfahren gemäß einem jeden der obigen Ansprüche, dadurch gekennzeichnet, daß man als Substratkomponente einen D-oder L-Aminosäureester verwendet, der aus Benzylestern oder geradkettigen oder verzweigten $C_1$-$C_6$-Alkylestern, die gegebenenfalls mit inerten Substituenten substituiert sind, ausgewählt ist.

10. Verfahren gemäß einem jeden der obigen Ansprüche, dadurch gekennzeichnet, daß man als nucleophile Komponente eine Aminosäure oder ein Aminosäureamid der Formeln

H-B-OH oder H-B-$NHR^3$

verwendet, in denen $R^3$ Wasserstoff oder $C_1$-$C_3$-Alkyl und B ein Aminosäurerest ist.

11. Verfahren nach Anspruch 1, dadurch gekennzeichnet, daß man als nucleophile Komponente einen Ester der Formel

H-B-$OR^4$

verwendet, in der B ein Aminocarbonsäurerest und $R^4$ $C_1$-$C_3$-Alkyl ist.

12. Verfahren gemäß einem jeden der obigen Ansprüche, dadurch gekennzeichnet, daß das sich ergebende Dipeptid eine C-terminale Schutzgruppe Y oder mehrere einschließt, und daß die Gruppe bzw. die Gruppen enzymatisch abgespalten wird bzw. werden, vorzugsweise mittels des gleichen Carboxypeptidaseenzyms, das in der vorgehenden Reaktion verwendet wurde.

13. Verfahren gemäß einem jeden der obigen Ansprüche, dadurch gekennzeichnet, daß das sich ergebende Dipeptid eine Seitenkettenschutzgruppe oder mehrere einschließt und daß die Gruppe bzw. die Gruppen enzymatisch gespalten wird bzw. werden, vorzugsweise mittels einer Esterase, einer Lipase oder eines proteolytischen Enzyms.

**Revendications**

1. Procédé pour produire des dipeptides ayant la formule générale

H-A-B-Y

dans laquelle A représente un résidu d'acide L- ou D-$\alpha$-aminé ou un résidu d'acide oméga-aminé,

éventuellement protégé en chaîne latérale, et B représente un résidu d'acide L- ou D-α-aminocarboxylique, éventuellement protégé en chaîne latérale, qui peut être identique à A ou différent de A, un résidu d'acide L- ou D-aminophosphonique ou encore un résidu d'acide L- ou D-aminosulfonique, ou bien l'acide oméga-aminé correspondant, ou un de leurs sels ou hydrates, et Y est OH ou un groupe bloquant C-terminal, caractérisé en ce qu'il consiste à faire réagir un composant substrat, ayant la formule

$H-A-OR^1$ ou 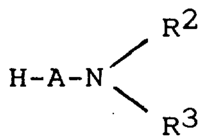

dans laquelle A est tel que défini ci-dessus, $R^1$ est un hydrogène ou un radical alkyle, aryle ou aralkyle éventuellement substitué par des substituants inertes, ou encore un fragment α-désamino d'un acide aminé, et $R^2$ et $R^3$ sont identiques ou différents et représentent chacun un hydrogène ou un radical alkyle, aryle ou aralkyle éventuellement substitué par des substituants inertes,

avec un composant nucléophile (qui, quand A = B, peut être formé in situ), choisi parmi :

(a) les acides aminés ayant la formule

H-B-OH

où B est un résidu d'acide aminocarboxylique tel que défini ci-dessus,

(b) les amides d'acides aminés ayant la formule

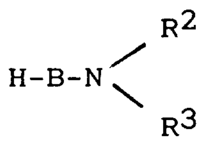

dans laquelle B est un résidu d'acide aminocarboxylique tel que défini ci-dessus, et $R^2$ et $R^3$ ont les significations données ci-dessus, sauf que, quand $R^2$ est un hydrogène, $R^3$ peut être un radical hydroxy ou amino,

(c) les esters d'acides L-aminés ayant la formule

$H-B-OR^4$

dans laquelle B est un résidu d'acide aminocarboxylique tel que défini ci-dessus, et $R^4$ est un radical alkyle, aryle ou aralkyle, et

(d) les acides aminophosphoniques ou aminosulfoniques à chaîne droite ou ramifiée, ayant la formule

$NH_2C_xH_zPO_3H_2$ ou $NH_2C_xH_zSO_3H$

où x vaut de 1-6 et z vaut de 2-12,

en présence d'une sérine ou de thiolcarboxypeptidase provenant d'une levure ou ayant une origine animale, végétale ou autrement microbienne, en solution ou suspension aqueuse ayant un pH de 5 à 10,5, et contenant éventuellement un solvant organique et/ou un sel, puis, si on le souhaite, à éliminer l'éventuel groupe protecteur en chaîne latérale ou le groupe protecteur Y et/ou, si on le souhaite, à convertir en un sel ou un hydrate le dérivé dipeptidique obtenu.

**2.** Procédé selon la revendication 1, caractérisé en ce qu'on utilise en tant que carboxypeptidase la carboxypeptidase Y provenant de la levure.

**3.** Procédé selon la revendication 2, caractérisé en ce que la carboxypeptidase utilisée a été purifiée par chromatographie d'affinité sur une résine d'affinité constituée d'un squelette de résine polymère comportant un certain nombre de groupes benzylsuccinyle couplés.

4. Procédé selon la revendication 1, caractérisé en ce que la carboxypeptidase utilisée est la pénicillocarboxypeptidase S-1 ou S-2 de Penicillium janthinellum, une carboxypeptidase d'Aspergillus saitoi ou d'Aspergillus oryzae, une carboxypeptidase C provenant de feuilles d'oranger ou de zeste d'orange, la carboxypeptidase $C_N$ provenant de Citrus natsudaidai Hayata, la phaséoline provenant des feuilles de haricot, ou encore une carboxypeptidase provenant de l'orge germée, du malt, de plants de coton germés, de tomates, de melons d'eau ou de poudre de broméléine (ananas).

5. Procédé selon les revendications 1 à 4, caractérisé en ce que la carboxypeptidase utilisée a subi une modification chimique ou encore est un mutant biosynthétique d'une forme naturelle.

6. Procédé selon l'une quelconque des revendications précédentes, caractérisé en ce que l'enzyme carboxypeptidase utilisée a été immobilisée.

7. Procédé selon l'une quelconque des revendications précédentes, caractérisé en ce qu'il consiste à utiliser une solution réactionnelle ou une dispersion réactionnelle aqueuse contenant de 0 à 70 % d'un solvant organique.

8. Procédé selon la revendication 7, caractérisé en ce que le solvant organique est choisi parmi les alcanols, le diméthylsulfoxyde, le diméthylformamide, le diméthoxyéthane, l'éthylèneglycol ou l'acétate d'éthyle.

9. Procédé selon l'une quelconque des revendications précédentes, caractérisé en ce qu'il consiste à utiliser comme composant substrat un ester d'un acide D- ou L-aminé choisi parmi les esters benzyliques ou les esters alkyliques en $C_1$-$C_6$ à chaîne droite ou ramifiée, éventuellement substitués par des substituants inertes.

10. Procédé selon l'une quelconque des revendications précédentes, caractérisé en ce qu'il consiste à utiliser comme composant nucléophile un acide aminé ou un amide d'acide aminé ayant la formule

H-B-OH ou H-B-$NHR^3$

où $R^3$ est un hydrogène ou un radical alkyle en $C_1$-$C_3$, et B est un résidu d'acide L-aminé.

11. Procédé selon la revendication 1, caractérisé en ce qu'il consiste à utiliser comme composant nucléophile un ester ayant la formule

H-B-$OR^4$

où B est un résidu d'acide aminocarboxylique, et $R^4$ est un radical alkyle en $C_1$-$C_3$.

12. Procédé selon l'une quelconque des revendications précédentes, caractérisé en ce que le dipeptide obtenu comprend un ou plusieurs groupes Y protecteurs C-terminaux et que le ou les groupes subissent un clivage enzymatique, de préférence au moyen de la même enzyme carboxypeptidase que celle qui avait été utilisée dans la réaction précédente.

13. Procédé selon l'une quelconque des revendications précédentes, caractérisé en ce que le dipeptide obtenu comprend un ou plusieurs groupes protecteurs en chaîne latérale, et que le ou les groupes subissent un clivage enzymatique, de préférence au moyen d'une estérase ou d'une lipase ou d'une enzyme protéolytique.

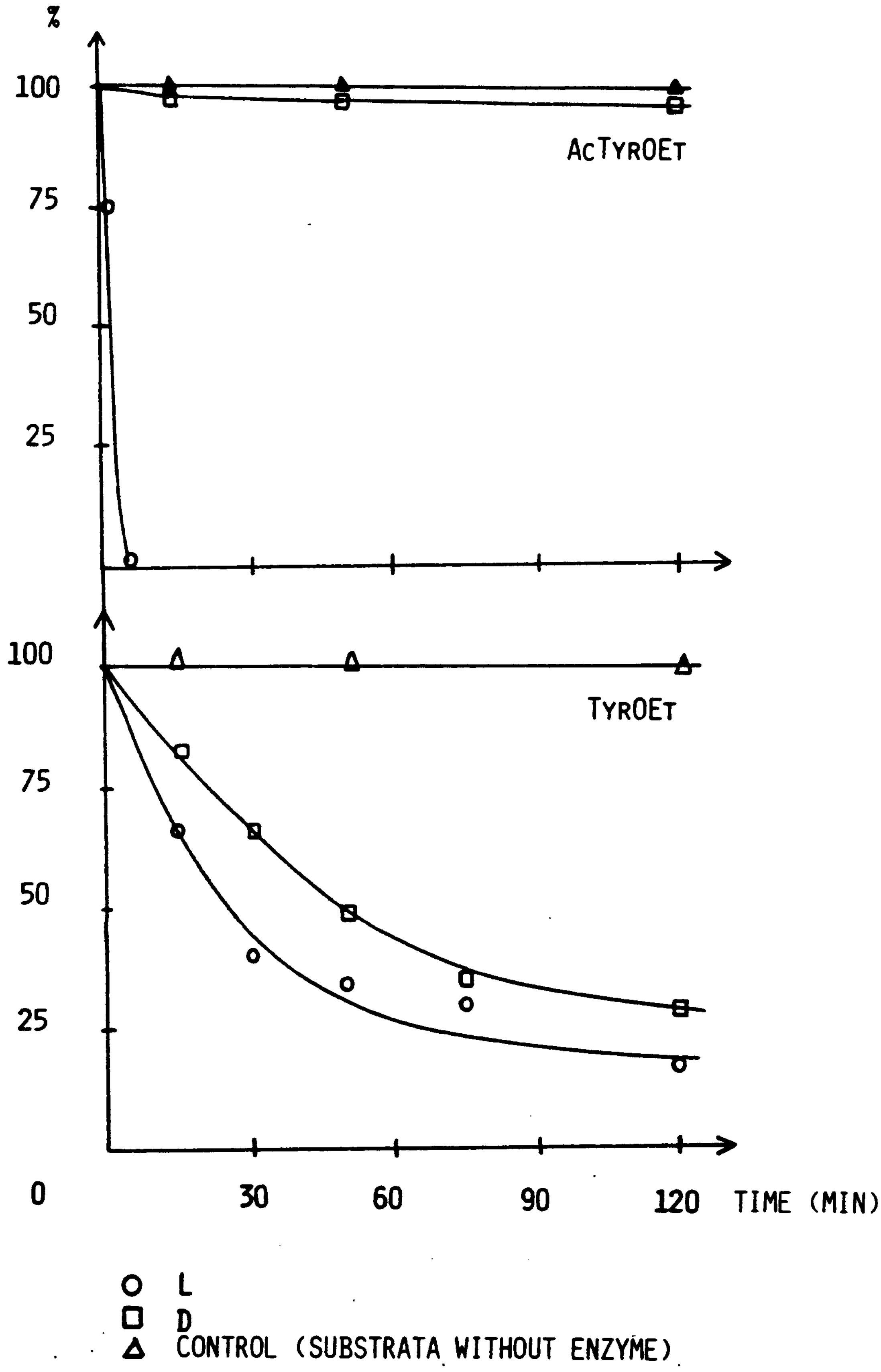


FIGURE 1

Carboxypeptidase Y (10 μM) Catalyzed Hydrolysis of L- and D-TyrOEt and of L- and D-AcTyrOEt (50 mM), 20% DMSO, pH 9,0, 1 mM EDTA

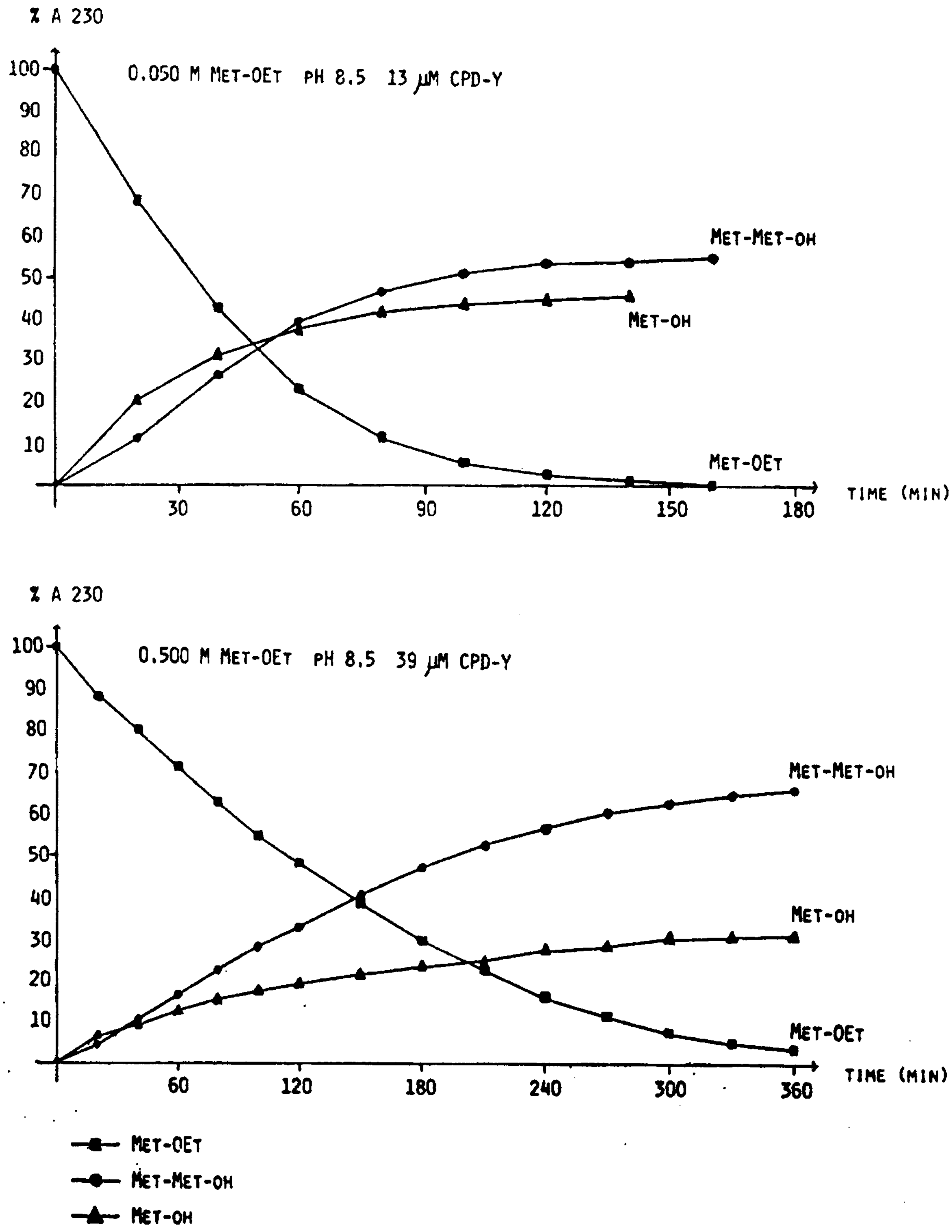


**Figure 2**

**Carboxypeptidase Y catalyzed formation of the homodipeptide L,L-Methionyl-Methionine from the single starting compound L-Methionine ethylester at 0.2 M and 0.5 M initial concentration, the nucleophile methionine being generated in situ. No oligomerisation is observed.**
**%A230 are per cent arbitrary absorbance units at 230 nm with HPLC-detection.**